# EUROPÄISCHE PATENTANMELDUNG

(11) **EP 2 100 554 A1**
(43) Veröffentlichungstag der Anmeldung: **16.09.2009**
(21) Anmeldenummer: 09003423.2
(22) Anmeldetag: 10.03.2009
(51) Int. Cl.: A61B 5/01, G01J 5/60

(54) **Verfahren und Vorrichtung zur Messung und Erzeugung von Kommunikation mittels Impulsen von wärmestrahlenden Körpern**

(30) Priorität: 15.03.2008 DE 102008014529
(71) Anmelder: Spechtmeyer, Horst-Wolfgang, 68782 Brühl (DE)
(72) Erfinder: Spechtmeyer, Horst-Wolfgang, 68782 Brühl (DE)

(57) **Zusammenfassung**

Zusammenfassung: Fig. 1 zeigt die Komponenten der Vorrichtung zur Messung und Erzeugung von Kommunikation mittels Impulsen von wärmestrahlenden Körpern. Die Wärmequelle E emittiert Infrarot-Impulse mit einer bevorzugten Frequenz. Der wärmestrahlende Körper R, der ebenfalls mit einer bevorzugten Frequenz sendet, empfängt einen Teil dieser Strahlung. Dabei entsteht unter bestimmten Voraussetzungen im Körper R eine Umwandlung der Strahlung. Die vom Körper R ausgehende Strahlung gelangt über einen Spiegel PAR zu einer Infrarot-Meß- und Analysiereinheit IR-MAU. Durch ein Magnetfeld kann die emittierende Strahlung des Körpers verändert werden. Auch die Art von E und R kann verändert werden; z. B. kann R der Kopf eines Menschen sein, der ein Gefühl hat und dadurch mit einer bevorzugten Frequenz abstrahlt. Das oben beschriebene Verfahren mit der in Fig. 1 dargestellten Vorrichtung ist eine einfache Methode, um auf einer höheren Stufe die Kommunikation zwischen IR-Impuls sendenden und empfangenden Körpern zu erzeugen, zu messen, zu analysieren und zu beweisen. Dadurch werden Erkenntnisse über das Gefühlsleben von Lebewesen, die Möglichkeit der Gefühlskommunikation von Robotern und den Einfluß der Strahlung von Himmelskörpern auf Lebewesen und Pflanzen gewonnen.

## Beschreibung

Es ist bekannt, dass Lebewesen oder andere wärmestrahlende Körper Infrarot-Impulse vorzugsweise im Bereich von 0,05 bis 10 Hz aussenden, die man messen (JP06339474) und einem bestimmten Gemütszustand zuordnen kann (DE 10 2006 014 143 A1).

Die im Patentanspruch 1 und 10 angegebene Erfindung befasst sich dagegen mit der Kommunikation zwischen wärmestrahlenden Körpern, die Infrarot-Impulse senden und empfangen.

Die in den Patentansprüchen 1 und 10 angegebene Erfindung sieht eine Vorrichtung bzw. ein Verfahren vor, bei der eine Strahlungsquelle Infrarot-Impulse mit einer dominierenden Frequenz oder in einem bevorzugten Frequenzbereich emittiert, und diese auf einen wärmestrahlenden Körper, der ebenfalls mit einer bevorzugten, möglicherweise jedoch anderen Frequenz emittiert, treffen. Dabei erfährt die Strahlung des Körpers eine Umwandlung.

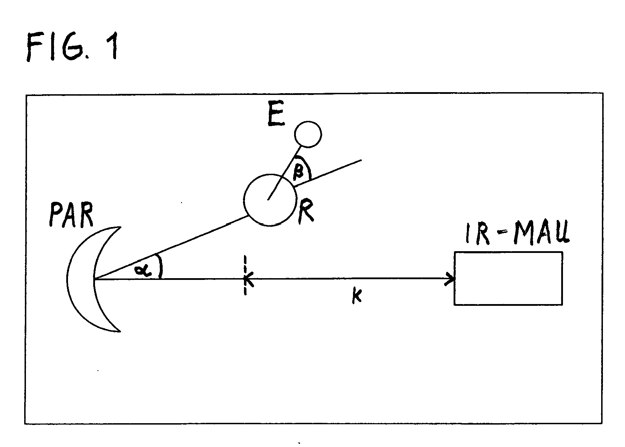

Die so erzeugte Strahlung wird über einen Spiegel PAR zu einer Infrarot-Meß- und Analysier-Einheit geleitet, die im Frequenzbereich von 0,05 bis 10 Hz mißt, um Einflüsse der Strahlungsquelle auf den Körper zu ermitteln. Eine mögliche Infrarot-Meß- und Analysier-Einheit ist in Fig. 1 der Offenlegungsschrift DE 10 2006 014 143 A1 dargestellt.

Hinsichtlich der geometrischen Anordnung ist nach Patentanspruch 2 und 3 vorgesehen, dass die Strahlungsquelle den Körper z. B. von hinten oder von der Seite bestrahlen kann oder dass sich die Infrarot-Meß- und Analysier-Einheit auf der Oberfläche des Körpers befindet.

Bei den Patentansprüchen 4, 5, 6 und 13 wird der wärmestrahlende Körper in ein Magnetfeld gebracht, um zusätzlich zur oder statt der Strahlungsumwandlung des Körpers durch die Strahlungsquelle eine weitere Veränderung der Strahlung des Körpers zu induzieren, wobei Stärke und Ausrichtung des Magnetfeldes verändert werden können.

Die Erfindung sieht nach den Patentansprüchen 7, 8 und 9 vor, dass z.B. der wärmestrahlende Körper ein Lebewesen ist, das durch Einstellung bestimmter Gefühle IR-Impulse mit unterschiedlicher Frequenz ausstrahlt, oder dass die Strahlungsquelle ein künstlich hergestellter IR-Impuls-Sender ist, dessen Impuls-Frequenzen variiert werden können, oder dass die Strahlungsquelle und der wärmestrahlende Körper Roboter sein können, die mittels IR-Impulsen auf höherer Stufe kommunizieren.

Das Verfahren zur Messung und Erzeugung von Kommunikation mittels Impulsen von wärmestrahlenden Körpern erfolgt nach Patentanspruch 11, 12 und 14 so, dass die Vorzugsimpulsfrequenz des Körpers zunächst konstant gehalten und nur die der Strahlungsquelle variiert wird und in weiteren Schritten das gleiche bei anderen Vorzugsimpulsfrequenzen durchgeführt wird. Die Vorzugsimpulsfrequenzen des Körpers können auch durch Gefühle oder durch Gefühlsvorstellungen von Lebewesen erzeugt werden. Es ist aber auch denkbar, dass Körper und Wärmequelle gleichzeitig Sender und Empfänger von IR-Impulsen sein können,so dass die Strahlung, die von der Wärmequelle bzw. von Wärmequelle und Körper ausgeht, untersucht werden kann.

Zusammenfassend kann man sagen, dass das oben beschriebene Verfahren mit der in Fig. 1 dargestellten Vorrichtung eine einfache Methode ist, um die Kommunikation zwischen IR-Impuls sendenden und empfangenden Körpern zu erzeugen, zu messen, zu analysieren und zu beweisen. Dadurch werden Erkenntnisse über das Gefühlsleben von Lebewesen, die Möglichkeit der IR-Kommunikation von Robotern und den Einfluß der Strahlung von Himmelskörpern auf Lebewesen und Pflanzen gewonnen.

Im folgenden sind Erfindungsbeispiele aufgeführt.

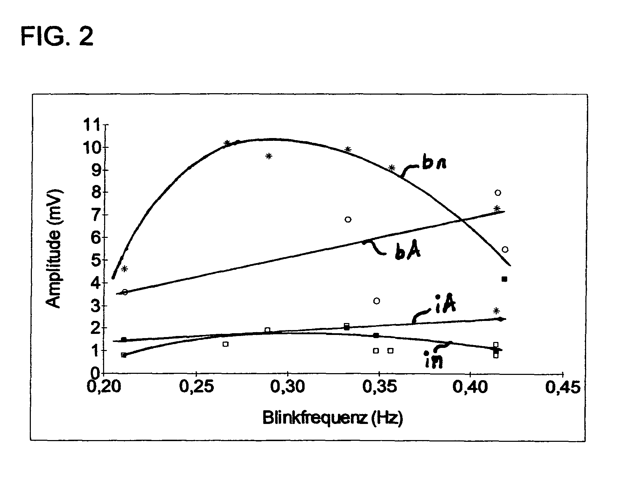

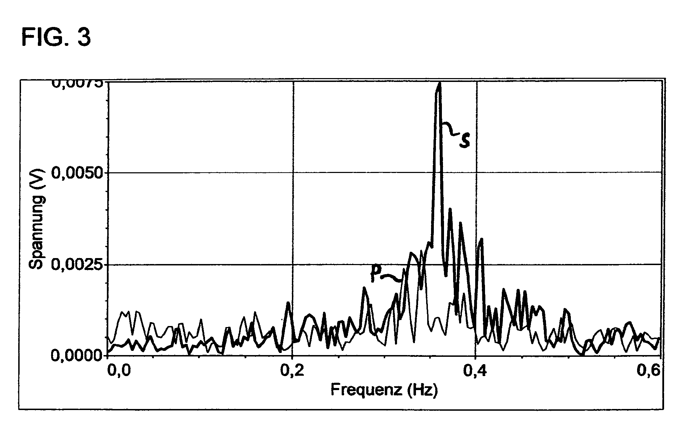

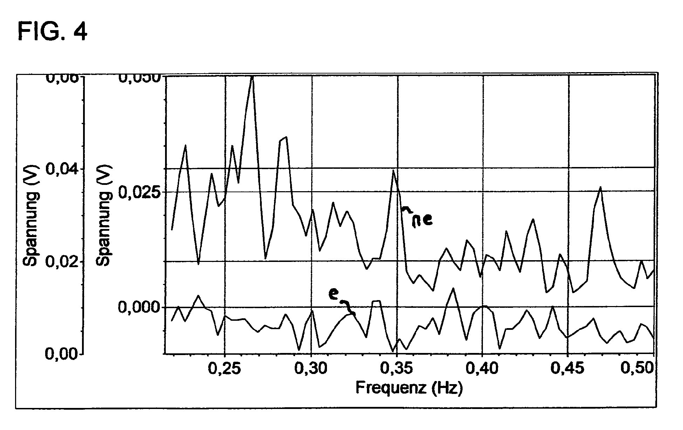

**Fig. 1** zeigt die Gesamtvorrichtung. In **Fig. 2** sind sowohl für die Gefühlsvorstellung "Amoroso" als auch für die Gefühlsvorstellung "Nichts" die Amplitude des Blinkpeaks und die des Vorstellungspeaks im Blinkfrequenzbereich zwischen 0,21 und 0,42 Hz aufgetragen. **Fig. 3** zeigt den Einfluß des Magnetfeldes. Die Mondfinsternis ist in **Fig. 4** ersichtlich.

**Fig. 1** zeigt die Komponenten der Vorrichtung zur Messung und Erzeugung von Kommunikation mittels Impulsen von wärmestrahlenden Körpern. Die Wärmequelle E emittiert Infrarot-Impulse mit einer bevorzugten Frequenz oder in einem bevorzugten Frequenzbereich. Der wärmestrahlende Körper R, der ebenfalls mit einer bevorzugten Frequenz bzw. in einem bevorzugten Frequenzbereich sendet, wobei Frequenz bzw. Frequenzbereich nicht wie bei der Wärmequelle sein müssen, empfängt einen Teil dieser Strahlung. Dabei entsteht unter bestimmten Voraussetzungen im Körper R eine Umwandlung der Strahlung. Die vom Körper R ausgehende Strahlung gelangt über einen Spiegel PAR zu einer Infrarot-Meß- und Analysiereinheit IR-MAU. Folgende Variationsmöglichkeiten sind vorgesehen:
Die Winkel α und β sowie die Länge können verändert werden, so dass z.B. bei α=β= =k=0 E, R, PAR und IR-MAU auf einer Achse liegen und sich IR-MAU auf der Oberfläche von R befindet. Bei konstanter Impulsfrequenz des Körpers wird die Impulsrequenz der Wärmequelle variiert und in weiteren Schritten erfolgt dasgleiche bei anderen Impulsrequenzen des Körpers. Durch ein Magnetfeld kann die emittierende Strahlung des Körpers verändert werden. Die Art von E und R kann verändert werden; z. B. kann R der Kopf eines Menschen sein, der ein Gefühl hat und dadurch mit einer bevorzugten Frequenz abstrahlt.
Bei den 3 Versuchen haben die Komponenten der Vorrichtung die im folgenden beschriebenen speziellen Eigenschaften.

Die Infrarot-Meß- und Analysiereinheit IR-MAU besteht aus einem Infrarot-Getektor, einem Verstärker, einer Analog-/Digital-Umwandlung, einem Analyseprogramm und einem Display. Der Infrarot-Detektor ist pyroelektrisch (PED).

### Technische Daten des PED-Detektors

Bandpass-Filter: 8 bis 14 µ; Durchmesser der Aktivfläche: 2 mm; Detektormaterial:
Lithium Tantalat; Empfindlichkeit bei Körpertemperatur 500 K und Chopper-Frequenz 1 Hz: 900 V/W; Verstärker-Spannung: 3-15 V; Arbeitsspannung: 6V.

Das Detektorelement ist zusammen dem Verstärker (60 dB bei 1 bis 1000 Hz) in einem Metallzylinder (ø 30 mm; Länge 90 mm) montiert.

Die verstärkten Signale (1 oder 2 Kanäle) gelangen mit geschirmten Leitungen, an deren Enden sich Klinkenstecker befinden, in einen Anschlusskasten aus Aluminium. Die Verbindungsleitung zwischen Anschlusskasten und Laptop ist mit einem 37-Pin-Stecker und einer Analog-/Digitalumwandlung (12-bit-A/D-Karte) abgeschlossen.

DieSampling Rate der Karte beträgt 0,006 Hz-100 kHz. Der Eingangsspannungsbereich beträgt ±10 V.

Es wird mit Software für das rechnergestützte Messen und Analysieren von Schwingungen, Lärm und Erschütterungen gearbeitet. Mit Hilfe der digitalen Signalanalyse (DAS) werden Diagramme von Zeitsignalen, Betragsspektren und Differenzspektren erstellt (Analyseprogramm).

Im Display kann man die Ergebnisse bei den unterschiedlichen Gemütszuständen des Meßobjekts sehen und vergleichen.

Beim 1. und 2. Versuch ist der Spiegel PAR ein Parabolspiegel. Die vom Gesicht R ausgestrahlte Wärme trifft auf einen mit Aluminium-Folie bedeckten Parabolspiegel PAR (810/730 mm), der sie auf den pyroelektrischen Infrarot-Detektor PED fokussiert. Das Gesicht, der Körper und die Arme wurden bewegungslos gehalten. Das menschliche Gesicht befand sich in einem Abstand von etwa 1,5 m zum Parabolspiegel. Die vom PAR reflektierte Wärmestrahlung wurde mit dem PED gemessen. Der Abstand vom PAR zum PED betrug 1,2 m. Als Meßparameter wurden bei allen 3 Versuchen ein Frequenzbereich von 100 Hz und eine Meßdauer von 256 s eingestellt.

Die Magnetplatte hatte die Abmessungen 120mmx75mmx1 mm und zeigte gegenüber einem Eisengewicht eine Haftkraft von 3 kp.

Die Strahlungsquelle E war eine 12V-5W-Glühbirne, die sich in einem Aluminium-Gehäuse befand und von einem Leistungsblinker für für 12V-Glüh- und Halogenlampen bis maximal 60 W gechoppt wurde. Die übrigen Eigenschaften befinden sich in der Beschreibung von Fig. 1.

### Versuch 1

Beim Versuch 1 strahlte die Glühbirne E seitlich auf das Gesicht R. Das Aluminium-Gehäuse befand sich 30 cm vom Gesicht und wies eine Öffnung von 10 cm x 10 cm auf. Zunächst wurde eine schläfrige, gelangweilte Einstellung eingenommen, so dass keine Emotionen geweckt wurden. Diese Einstellung wird im folgenden "Nichts" genannt. Es wurde eine diskrete Blinkfrequenz im Bereich zwischen 0,21 und 0,42 Hz eingestellt und eine 256 s dauernde Messung durchgeführt. Das Frequenzspektrum der Messung zeigt den Peak der Blinkfrequenz und einen Peak in einem Frequenzbereich, die für die Vorstellung "Nichts" typisch ist. Für verschiedene Blinkfrequenzen im Frequenzbereich 0,21 bis 0,42 Hz wurden Messungen durchgeführt. Dabei wurde auch als konträres Gefühl die Vorstellung "Amoroso" angenommen.

**Fig.2** zeigt den Frequenzverlauf der Amplituden der Blink-(bn) und

Vorstellungspeaks (in) beim Gefühl "Nichts" und den Frequenzverlauf der Amplituden der Blink-(bA) und Vorstellungspeaks(iA) beim Gefühl "Amoroso". Sowohl die Vorstellungs- als auch die Blinkamplituden zeigen, dass bei "Nichts" die höchste Amplitude bei 0,30 Hz und bei "Amoroso" bei 0,42 Hz liegt. Normalerweise würde die Blinkamplitude bei fehlendem Gesicht R von 0,21 Hz auf 0,42 Hz kontinuierlich um etwa 8 mV fallen. Die obigen Ergebnisse sagen aus, dass eine Einflussnahme von E auf R und damit eine Umwandlung des Strahlungsspektrums von R durch E erfolgt.

### Versuch 2

Versuch 2 diente dazu, den Einfluss eines Magnetfeldes auf das Gesicht R zu unter suchen. Um den doppelten Einfluß von blinkender Strahlungsquelle E und eines Magnetfeldes zu vermeiden, wurde bei diesem Versuch auf E verzichtet. Die oben beschriebene Magnetplatte wurde in 2 senkrecht zueinander stehenden Richtungen erprobt: parallel zur Stirn und direkt oberhalb der Stirn bzw. senkrecht zur Stirn auf dem Kopf.

**Fig. 3** zeigt die Frequenzspektren der Magnetplatte in paralleler (p) und senkrechter (s) Anordnung für das Gefühl "Amoroso". Bei paralleler Anordnung betrug die höchste Spannung nur 2,9 mV (0,34 Hz), dagegen bei senkrechter Anordnung 7,4 mV (0,36 Hz). Bei vertikaler Ausrichtung der Empfänger im Kopf kann sich offenbar ein größeres Gefühl "Amoroso" ausbilden.

### Versuch 3

Beim Versuch 3 wurde die Mondfinsternis untersucht. Dabei war die
Strahlungsquelle E die Sonne, der wärmestrahlende Körper R die Erde und der Spiegel PAR der Mond. Der Versuch wurde durchgeführt, um etwas über die Erde zu erfahren. Dabei waren α=β=k=0; d. h. die Infrarot-Meß- und Analysier-Einheit IR-MAU befand sich auf der Erdoberfläche. Die Kommunikation der Sonne mit der Erde ergab die vom Mond reflektierte IR-Strahlung.

**Fig. 4** zeigt die Frequenzspektren im Bereich von 0,215 bis 0,5 Hz, wenn der Mond sich noch nicht im Erdschatten (ne) bzw. wenn er sich im Erdschatten (e) befindet. Im Fall des Erdschattens liegt im untersuchten Frequenzbereich der höchste Peak bei 0,383 Hz (14,2 mV); im anderen Fall liegen die höchsten Peaks bei niedrigeren Frequenzen.

## Patentansprüche

1. Vorrichtung zur Messung und Erzeugung von Kommunikation mittels Impulsen von wärmestrahlenden Körpern entsprechend Fig. 1, wobei von einer Strahlungsquelle E Infrarot-Impulse mit einer dominierenden Frequenz emittiert werden, diese auf einen wärmestrahlenden Körper R, der ebenfalls mit einer bevorzugten, möglicherweise jedoch anderen Frequenz emittiert, treffen, die dabei erzeugten Strahlen zumindestens teilweise auf einen Spiegel PAR gelangen und mit einer Infrarot-Meß- und Analysier-Einheit IR-MAU im Frequenzbereich von 0,05 bis 10 Hz untersucht werden.

2. Vorrichtung nach Anspruch 1, wobei in der geometrischen Anordnung der Fig. 1 die Winkel α und β vorgesehen sind, die variieren können, aber auch Null sein können.

3. Vorrichtung nach Anspruch 1 und 2, wobei in der geometrischen Anordnung der Fig. 1 die Länge k vorgesehen ist, die variieren kann, aber auch Null sein kann, so dass sich bei α=β=k=0 die Infrarot Meß- und Analysier-Einheit IR-MAU auf der Oberfläche von R befindet.

4. Vorrichtung nach Anspruch 1,2 und 3, wobei der wärmestrahlende Körper R sich in einem Magnetfeld befindet.

5. Vorrichtung nach 4, wobei das Magnetfeld von einer Magnetplatte erzeugt wird, die hinsichtlich Stärke und Ausrichtung variiert werden kann.

6. Vorrichtung nach mindestens einem der Patentansprüche 1 bis 5, wobei anstelle der Strahlungsquelle E ein Magnetfeld der in Anspruch 4 bzw. 5 beschriebenen Art auf den wärmestrahlenden Körper R einwirkt.

7. Vorrichtung nach mindestens einem der Patentansprüche 1 bis 6, wobei der wärmestrahlende Körper R ein Lebewesen ist, das durch Einstellung bestimmter Gefühle IR-Impulse mit unterschiedlicher Frequenz ausstrahlt.

8. Vorrichtung nach mindestens einem der Patentansprüche 1 bis 5 und 7, wobei die Strahlungsquelle E ein künstlich hergestellter IR-Impuls-Sender ist, dessen Impuls-Frequenzen variiert werden können.

9. Vorrichtung nach mindestens einem der Patentansprüche 1 bis 5 und 7 bis 8, wobei die Strahlungsquelle E und der wärmestrahlende Körper R Roboter sein können, die mittels IR-Impulsen kommunizieren.

10. Verfahren zur Messung und Erzeugung von Kommunikation mittels Impulsen von wärmestrahlenden Körpern, wobei ein wärmestrahlender Körper **dadurch** zur Messung der Kommunikation vorbereitet bzw. zur Erzeugung der Kommunikation angeregt wird, dass ein Infrarot-Impuls einer Wärmequelle, die mit einer bevorzugten Frequenz emittiert, auf den Körper strahlt, der ebenfalls eine bevorzugte, möglicherweise jedoch andere IR-Impuls-Frequenz aufweist, wodurch eine Änderung der Wärmestrahlung des Körpers entsteht, die gemessen und analysiert wird, um Eigenschaften des wärmestrahlenden Körpers bzw. Einflüsse der Wärmequelle auf den wärmestrahlenden Körper zu ermitteln.

11. Verfahren nach Patentanspruch 10, wobei die Vorzugsimpulsfrequenz des Körpers zunächst konstant gehalten und nur die der Strahlungsquelle variiert wird wird und in weiteren Schritten das gleiche bei anderen Vorzugsimputsfrequenzen durchgeführt wird.

12. Verfahren nach Patentanspruch 10 und 11, wobei die Vorzugsimpulsfrequenzen des Körpers durch Gefühle oder durch Gefühlsvorstellungen von Lebewesen erzeugt werden.

13. Verfahren nach mindestens einem der Patentansprüche 10 bis 12, wobei der wärmestrahlende Körper in ein Magnetfeld gebracht wird, das zusätzlich zur Wärmequelle oder anstelle der Wärmequelle so wirkt, dass eine Veränderung der Körperstrahlung auftritt.

14. Verfahren nach mindestens einem der Patentansprüche 10 bis 13, wobei der Körper und die Wärmequelle Sender und Empfänger von IR-Impulsen sind und die Strahlung, die von der Wärmequelle bzw. von Wärmequelle und Körper ausgeht, untersucht wird.

## Geänderte Patentansprüche

### Geänderte Patentansprüche gemäss Regel 137(2) EPÜ.

**1.** Vorrichtung zur Messung und Erzeugung von Kommunikation mittels Impulsen von wärmestrahlenden Körpern, wobei von einer Strahlungsquelle E Infrarot-Impulse mit einer dominierenden Frequenz emittiert werden, diese auf einen wärmestrahlenden Körper R, der ebenfalls mit einer bevorzugten, möglicherweise jedoch anderen Frequenz emittiert, treffen, die dabei erzeugten Strahlen zumindestens teilweise auf einen Spiegel PAR gelangen und mit einer Infrarot-Meß- und Analysier-Einheit IR-MAU im Frequenzbereich von 0,05 bis 10 Hz untersucht werden.

**2.** Vorrichtung nach Anspruch 1, wobei der Winkel zwischen dem auf den Spiegel einfallendem Strahl und dem vom Spiegel reflektierten Strahl, der Winkel zwischen dem auf den Spiegel bzw. von der Wärmequelle auf den Körper einfallenden Strahl sowie die Abstände zwischen Wärmequelle, Körper, Spiegel und Infrarot-Mess- und Analysier-Einheit variabel sind.

**3.** Vorrichtung nach Anspruch 2, wobei die Infrarot-Mess- und Analysier-Einheit sich auf dem Körper auf der dem Spiegel zugewandten Seite und die Wärmequelle sich auf der Gegenseite befindet.

**4.** Vorrichtung nach Anspruch 1,2 und 3, wobei der wärmestrahlende Körper R sich in einem Magnetfeld befindet.

**5.** Vorrichtung nach 4, wobei das Magnetfeld von einer Magnetplatte erzeugt wird und die Feldstärke der Magnetplatte und Ausrichtung der Magnetplatte bezüglich des wärmestrahlenden Körpers R variiert werden können.

**6.** Vorrichtung nach mindestens einem der Patentansprüche 1 bis 5, wobei anstelle der Strahlungsquelle E ein Magnetfeld der in Anspruch 4 bzw. 5 beschriebenen Art auf den wärmestrahlenden Körper R einwirkt.

**7.** Vorrichtung nach mindestens einem der Patentansprüche 1 bis 6, wobei der wärmestrahlende Körper R ein Lebewesen ist, das durch Einstellung bestimmter Gefühle IR-Impulse mit unterschiedlicher Frequenz ausstrahlt.

**8.** Vorrichtung nach mindestens einem der Patentansprüche 1 bis 5 und 7, wobei die Strahlungsquelle E ein künstlich hergestellter IR-Impuls-Sender ist, dessen Impuls-Frequenzen variiert werden können.

**9.** Vorrichtung nach mindestens einem der Patentansprüche 1 bis 5 und 7 bis 8, wobei die Strahlungsquelle E und der wärmestrahlende Körper R Roboter sind, die mittels IR-Impulsen kommunizieren.

**10.** Verfahren zur Messung und Erzeugung von Kommunikation mittels Impulsen von wärmestrahlenden Körpern, wobei ein wärmestrahlender Körper **dadurch** zur Messung der Kommunikation vorbereitet bzw. zur Erzeugung der Kommunikation angeregt wird, dass ein Infrarot-Impuls einer Wärmequelle, die mit einer bevorzugten Frequenz emittiert, auf den Körper strahlt, der ebenfalls eine bevorzugte, möglicherweise jedoch andere IR-Impuls-Frequenz aufweist, wodurch eine Änderung der Wärmestrahlung des Körpers entsteht, die gemessen und analysiert wird, um Eigenschaften des wärmestrahlenden Körpers bzw. Einflüsse der Wärmequelle auf den wärmestrahlenden Körper zu ermitteln.

**11.** Verfahren nach Patentanspruch 10, wobei die Vorzugsimpulsfrequenz des Körpers zunächst konstant gehalten und nur die der Strahlungsquelle variiert wird wird und in weiteren Schritten das gleiche bei anderen Vorzugsimpulsfrequenzen durchgeführt wird.

**12.** Verfahren nach Patentanspruch 10 und 11, wobei die Vorzugsimpulsfrequenzen des Körpers durch Gefühle oder durch Gefühlsvorstellungen von Lebewesen erzeugt werden.

**13.** Verfahren nach mindestens einem der Patentansprüche 10 bis 12, wobei der wärmestrahlende Körper in ein Magnetfeld gebracht wird, das zusätzlich zur Wärmequelle so wirkt, dass eine Veränderung der Körperstrahlung auftritt.

**14.** Verfahren nach mindestens einem der Patentansprüche 10 bis 13, wobei der Körper und die Wärmequelle Sender und Empfänger von IR-Impulsen sind und die Strahlung, die von der Wärmequelle bzw. von Wärmequelle und Körper ausgeht, untersucht wird.

**15.** Verfahren zur Beeinflussung der Wärmestrahlung eines Körpers, wobei der wärmestrahlende Körper in ein Magnetfeld gebracht wird und die Infrarot-Strahlen über einen Parabolspiegel zur Mess- und Analysier-Einheit gelangen.
